# EUROPEAN PATENT SPECIFICATION

(11) **EP 0 298 732 B1**
(45) Date of publication and mention of the grant of the patent: **01.09.1993**
(21) Application number: 88306188.9
(22) Date of filing: 07.07.1988
(51) Int. Cl.: C07K 7/26, A61K 37/02

(54) **Therapeutic Somatostatin analogues**
Therapeutische Analoga von Somatostatin
Analogues thérapeutiques de somatostatine

(30) Priority: 07.07.1987 US 70400
(43) Date of publication of application: 11.01.1989
(73) Proprietor: The Administrators of The Tulane Educational Fund, New Orleans Louisiana 70112 (US)
(72) Inventor: Coy, David H., New Orleans Louisiana 70115 (US); Murphy, William A., Covington Louisiana 70433 (US); Heiman, Mark L., New Orleans Louisiana 70122 (US)
(74) Representative: Sheard, Andrew Gregory

(56) References cited:
- EP-A- 0 175 644
- EP-A- 0 215 171
- EP-A- 0 222 578
- US-A- 4 328 135
- PROCEEDINGS OF THE NATIONAL ACADEMY OF SCIENCES, USA, vol. 83, 1986, pages 1896-1900; R.Z. CAI et al.: "Synthesis and bilogical activity of highly potent octapeptide analogs of somatostatin"
- IDEM

## Description

This invention relates to a therapeutic peptide.

R Z Cai *et al, Proc. Natl. Acad. Sci. USA* **83**: 1896-1900 (March 1986) relates to octapeptide analogues of somatostatin as does EP-A-0215171.

A number of somatostatin analogues exhibiting GH-release-inhibiting activity have been described in the literature, including analogues containing fewer than the naturally occurring fourteen amino acids. For example, Coy et al, US Patent No. 4485101, describes dodecapeptides having an N-terminal acetyl group, a C-terminal NH₂, D-Trp at position 6, and p-C1-Phe at position 4. (Herein, when no designation of configuration is given, the L-isomer is intended.)

Therefore, according to a first aspect of the invention there is provided a peptide characterised in that it comprises the sequence

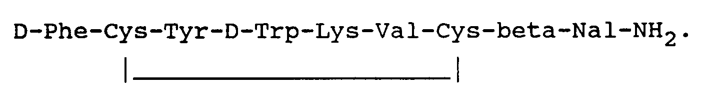
D-Phe-Cys-Tyr-D-Trp-Lys-Val-Cys-beta-Nal-NH2.


In this specification β-Nal means β-naphthylalanine.

A second aspect of the invention relates to a peptide of the first aspect for use in medicine.

Thus, a third aspect relates to the use of a peptide of the first aspect in the preparation of a therapeutic agent. Such therapeutic activity includes the inhibition of release or secretion of GH, insulin, glucagon, the treatment of cancer, acromegaly, ulcers, pancreatitis, diarrhoea, diabetes, cirrhosis, Alzheimer's disease, mushroom poisoning or pain (ie as an analgesic).

A fourth aspect of the present invention relates to a pharmaceutical composition comprising a peptide of the first aspect in admixture with a pharmaceutically acceptable carrier.

The invention thus encompasses, in its fifth aspect, a process for the preparation of a pharmaceutical composition comprising admixing a peptide of the first aspect with a pharmaceutically acceptable carrier.

In a sixth aspect of the present invention there is provided a process for the preparation of a peptide of the first aspect, the process comprising coupling together successive amino acids.

In preferred embodiments, a therapeutically effective amount of the therapeutic compound is combined with a pharmaceutically acceptable carrier substance (eg, mannitol, magnesium carbonate, lactose, an emulsion or dispersion in a lipophilic substance such as pamoic acid, or a phospholipid with which the therapeutic compound can form a micelle). The most preferred carrier substance is mannitol. Examples of such compositions, include a pill, tablet, capsule, or liquid for oral administration to a human patient, a spreadable cream, gel, lotion, or ointment for application to the skin of a human patient in need of the compound, a liquid capable of being administered nasally as drops or spray, or a liquid capable of intravenous, parenteral, subcutaneous, or intraperitoneal administration. The pill, tablet or capsule can be coated with a substance capable of protecting the composition from the gastric acid in the patient's stomach for a period of time sufficient to allow the composition to pass undisintegrated into the patient's small intestine. The therapeutic composition can also be administered in the form of an oil emulsion or dispersion in conjunction with a lipophilic salt such as pamoic acid. The therapeutic composition can also be in the form of a biodegradable sustained release formulation for intramuscular administration. For maximum efficacy, zero order release is desired. Zero order release can be obtained using an implantable or external pump, eg, INFUSAID (trade mark) pump, to administer the therapeutic composition.

The compound of the invention is active in inhibiting the secretion of GH, insulin, and glucagon. Further, the aromatic lipophilic N-terminal end can provide long-lasting in vivo activity.

Other features and advantages of the invention will be apparent from the following description of the preferred embodiments thereof, and from the claims.

The octapeptide of the invention may be an analogue of somatostatin which has D-Trp at position 4, Lys at position 5, Val at position 6, and Cys at positions 2 and 7; the presence of a Cys residue near each end of the peptide results in a cyclized molecule.

The compound can be provided in the form of a pharmaceutically acceptable salt. Examples of preferred salts are those with therapeutically acceptable organic acids, eg, acetic, lactic, maleic, citric, malic, ascorbic, succinic, benzoic, salicylic, methanesulphonic, toluenesulphonic, or pamoic acid, as well as polymeric acids such as tannic acid or carboxymethyl cellulose, and salts with inorganic acids such as the hydrohalic acids, eg, hydrochloric acid, sulphuric acid, or phosphoric acid.

### Comparative Example 1

The synthesis of one octapeptide follows. The octapeptide of the invention can be prepared by making appropriate modifications, within the ability of someone of ordinary skill in this field, of the following synthetic method.

The first step in the preparation of 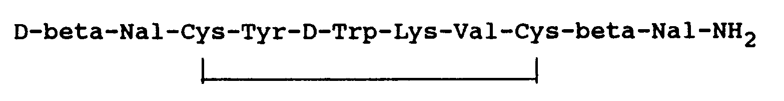
D-beta-Nal-Cys-Tyr-D-Trp-Lys-Val-Cys-beta-Nal-NH2

was the preparation of the intermediate tert-butyloxycarbonyl-D-beta-Nal-S-methylbenzyl-Cys-Tyr-D-Trp-N^{e}-benzyloxycarbonyl-Lys-Val-S-methylbenzyl-Cys-O-benzyl-beta-Nal-benzyhydrylaminine resin, as follows.

Benzhydrylamine-polystyrene resin (Vega Biochemicals, Inc) in the chloride ion form was placed in the reaction vessel of a Beckman 990B peptide synthesizer programmed to perform the following reaction cycle: (a) methylene chloride; (b) 33% trifluoroacetic acid in methylene chloride (2 times for 1 and 25 min each); (c) methylene chloride; (d) ethanol; (e) methylene chloride; (f) 10% triethylamine in chloroform.

The neutralized resin was stirred with Boc-O-benzyl-beta-Nal and diisopropylcarbodiimide (1.5 mmole each) in methylene chloride for 1 hr and the resulting amino acid resin was then cycled through steps (a) to (g) in the above wash program. In this specification 'Boc' means butyl oxycarbonyl. The following amino acids (1.5 mmole) were then coupled successively by the same procedure; Boc-S-methylbenzyl-Cys, Boc-Val, Boc-Ne-benzyloxycarbonyl-lysine, Boc-D-Trp, Boc-Tyr, Boc-S-methylbenzyl-Cys, Boc-D-beta-Napthylalanine.

The resin was washed and dried and then mixed with anisole (4 ml) and anhydrous hydrogen fluoride (36 ml) at 0°C and stirred for 45 min. (one can also use thioanisole, trifluoroacetic acid, and trifluoromethane sulphonic acid at a ratio of 1:90:9, for 6hrs). Excess hydrogen fluoride was evaporated rapidly under a stream of dry nitrogen and free peptide precipitated and washed with ether. The crude peptide was then dissolved in 800 ml of 90% acetic acid to which was added iodine in methanol until a permanent brown colour was present. The solution was then stirred for 1 hr before removing the solvent in vacuo. The resulting oil was dissolved in a minimum volume of 50% acetic acid and eluted on a column (2.5 x 100 mm) of Sephadex G-25. Fractions containing a major component by uv absorption and thin layer chromatography were then pooled, evaporated to a small volume, and applied to a column (2.5 x 50 cm) of Whatman LRP-1 octadecylsilane (15-20 uM).

The column was eluted with a linear gradient of 10-50% acetonitrile in 0.1% trifluoroacetic acid in water. Fractions were examined by thin layer chromatography and analytical high performance liquid chromatography and pooled to give maximum purity and if desired, a different salt prepared, eg, acetate or phosphate. Repeated lyophilization of the solution from water gave 170 mg of the product as a white, fluffy powder.

The product was found to be homogeneous by Hplc (high pressure liquid chromatography) and Tlc (thin layer chromatography). Amino acid analysis of an acid hydrolysate confirmed the composition of the octapeptide.

The octapeptide of the invention having the formula 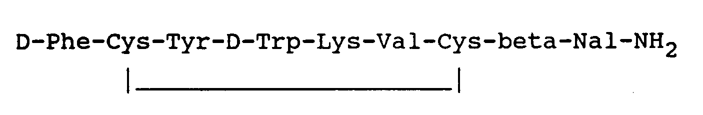
D-Phe-Cys-Tyr-D-Trp-Lys-Val-Cys-beta-Nal-NH2
was made according to a method analogous to that described above.

When administered to mammals, particularly humans, (eg orally, topically, intravenously, parenterally in a sustained release, biodegradable form, nasally, or by suppository), the compound can be effective to inhibit GH release as well as to inhibit insulin, glucagon, and pancreatic exocrine secretion, and to therapeutically affect the central nervous system.

The compound can be administered to a mammal, eg a human, in the dosages used for somatostatin or, because of its greater potency, in smaller dosages. The compound of the invention can be used for the treatment of cancer, particularly growth hormone-dependent cancer (eg, bone, cartilage, pancreas (endocrine and exocrine), prostate, or breast), acromegaly and related hypersecretory endocrine states, or of bleeding ulcers in emergency patients and in those suffering from pancreatitis or diarrhoea. The compound can also be used in the management of diabetes and to protect the liver of patients suffering from cirrhosis or hepatitis. The compound can also be used to treat Alzheimer's disease, as analgesics to treat pain by acting specifically on certain opiate receptors, and as gastric cytoprotective compound for ulcer therapy. The compound can also be used to treat certain types of mushroom poisoning.

The compound can also be used to treat diabetes-related retinopathy. The anti-cancer activity of the compound may be related to its ability to antagonize cancer-related growth factors such as epidermal growth factor.

The compound can be administered to a mammal, eg, a human, in a dosage of 0.01 to 1000 µg/kg/day, preferably 0.1 to 100 µg/kg/day.

## Claims (Claims for the following Contracting State(s): AT, BE, CH, DE, FR, GB, IT, LI, LU, NL, SE)

1. An octapeptide of the formula 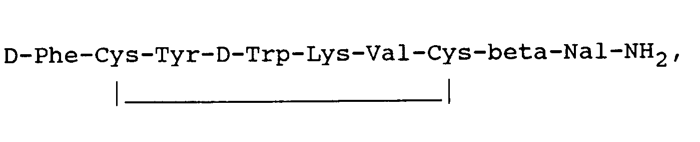
D-Phe-Cys-Tyr-D-Trp-Lys-Val-Cys-beta-Nal-NH2,
 or a pharmaceutically acceptable salt thereof.

2. An octapeptide of the formula D-Phe-Cys-Tyr-D-Trp-Lys-Val-Cys-beta-Nal-NH2, or a pharmaceutically acceptable salt thereof, for use in medicine.

3. The use of an octapeptide of the formula 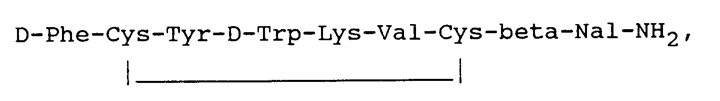
D-Phe-Cys-Tyr-D-Trp-Lys-Val-Cys-beta-Nal-NH2,

 in the preparation of a therapeutic agent.

4. The use as claimed in claim 3 wherein the therapeutic activity is the inhibition of release or secretion of GH, insulin, glucagon, treatment of cancer, acromegaly, ulcers, pancreatitis, diarrhoea, diabetes, cirrhosis, Alzheimer's disease, mushroom poisoning or pain as an analgesic.

5. A pharmaceutical composition comprising an octapeptide of the formula 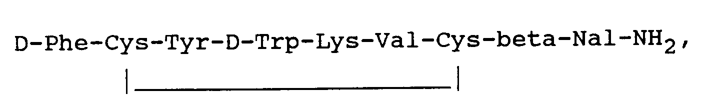
D-Phe-Cys-Tyr-D-Trp-Lys-Val-Cys-beta-Nal-NH2,
 or a pharmaceutically acceptable salt thereof, in admixture with a pharmaceutically acceptable carrier.

## Claims (Claims for the following Contracting State(s): ES, GR)

1. A process for the preparation of an octapeptide of the formula 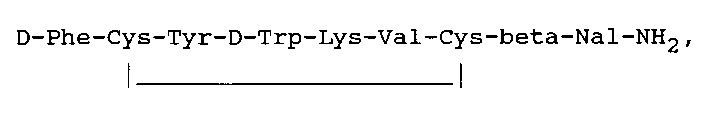
D-Phe-Cys-Tyr-D-Trp-Lys-Val-Cys-beta-Nal-NH2,
 or a pharmaceutically acceptable salt thereof, the process comprising coupling together successive amino acids.

2. A process for the preparation of a pharmaceutical composition, the process comprising admixing an octapeptide of the general formula 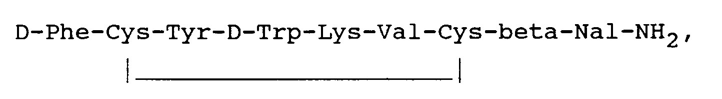
D-Phe-Cys-Tyr-D-Trp-Lys-Val-Cys-beta-Nal-NH2,
 or a pharmaceutically acceptable salt thereof, with a pharmaceutically acceptable carrier.

3. The use of an octapeptide of the formula D-Phe-Cys-Tyr-D-Trp-Lys-Val-Cys-beta-Nal-NH2, or a pharmaceutically acceptable salt thereof, in the preparation of a therapeutic agent.

4. The use as claimed in claim 3 wherein the therapeutic activity is the inhibition of release or secretion of GH, insulin, glucagon, treatment of cancer, acromegaly, ulcers, pancreatitis, diarrhoea, diabetes, cirrhosis, Alzheimer's disease, mushroom poisoning or pain as an analgesic.

## Patentansprüche (Patentansprüche für folgende(n) Vertragsstaat(en): AT, BE, CH, DE, FR, GB, IT, LI, LU, NL, SE)

1. Ein Octapeptid der Formel 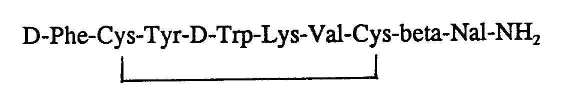
D-Phe-Cys-Tyr-D-Trp-Lys-Val-Cys-beta-Nal-NH2

 oder ein pharmazeutisch annehmbares Salz davon.

2. Ein Octapeptid der Formel 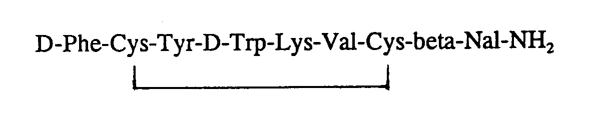
D-Phe-Cys-Tyr-D-Trp-Lys-Val-Cys-beta-Nal-NH2

 oder ein pharmazeutisch annehmbares Salz davon, für die Verwendung in der Medizin.

3. Verwendung eines Octapeptids der Formel 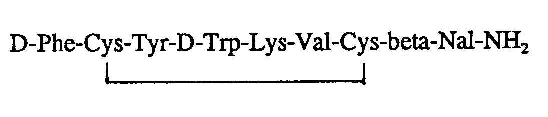
D-Phe-Cys-Tyr-D-Trp-Lys-Val-Cys-beta-Nal-NH2

 bei der Herstellung eines therapeutischen Wirkstoffes.

4. Verwendung nach Anspruch 3, wobei die therapeutische Aktivität die Hinderung der Freisetzung oder Sekretion von GH, Insulin Glucagon, die Behandlung von Krebs, Acromegalie, Geschwüren, Pankreatitis, Diarrhöe", Diabetes, Zirrhose, Alzheimer'sche Krankheit, Pilzvergiftung oder Schmerzen als Analgetikum ist..

5. Pharmazeutische Zusammensetzung, die ein Octapeptid der Formel 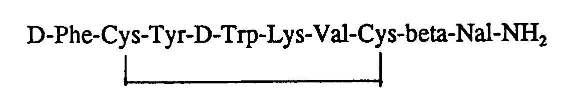
D-Phe-Cys-Tyr-D-Trp-Lys-Val-Cys-beta-Nal-NH2
 oder ein phamazeutisch annehmbares Salz davon in Mischung mit einem pharmazeutisch annehmbaren Träger enthält.

## Patentansprüche (Patentansprüche für folgende(n) Vertragsstaat(en): ES, GR)

1. Verfahren zur Herstellung eines Octapeptis der Formel 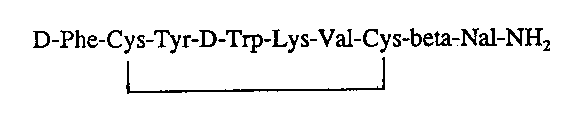
D-Phe-Cys-Tyr-D-Trp-Lys-Val-Cys-beta-Nal-NH2

 oder eines pharmazeutische annehmbaren Salzes davon, welches Verfahren das Zusammenfügen aufeinanderfolgender Aminosäuren umfaßt.

2. Verfahren zur Herstellung einer pharmazeutischen Zusammensetzung, welches das Zusammenmischen eines Octapeptids der allgemeinen Formel 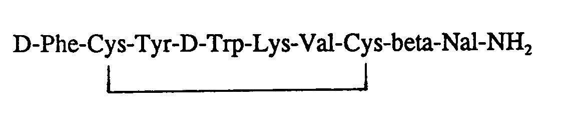
D-Phe-Cys-Tyr-D-Trp-Lys-Val-Cys-beta-Nal-NH2
 oder eines pharmazeutisch annehmbaren Salzes davon mit einem pharmazeutisch annembaren Trgäger umfaßt.

3. Verwendung eines Octapeptids der Formel 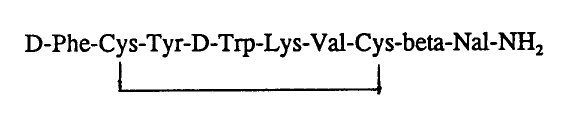
D-Phe-Cys-Tyr-D-Trp-Lys-Val-Cys-beta-Nal-NH2

 oder eines pharmazeutisch annehmbaren Salzes davon bei der Herstellung eines therapeutischen Wirkstoffes.

4. Verwendung nach Anspruch 3, bei welchem die therapeutische Aktivität die Hinderung der Freisetzung oder der Sekretion von GH, Insulin, Glucagon, die Behandlung von Krebs, Acromegalie, Geschwüren, Pankreatitis, Diarrhöe", Diabetes, Zirrhose, Alzheimer'sche Krankheit, Pilzvergiftung oder Schmerzen als Analgetikum ist.

## Revendications (Revendications pour l'(les) Etat(s) contractant(s) suivant(s): AT, BE, CH, DE, FR, GB, IT, LI, LU, NL, SE)

1. Octapeptide de formule D-Phé-Cys-Tyr-D-Trp-Lys-Val-Cys-bêta-Nal-NH2, ou un de ses sels pharmaceutiquement acceptables.

2. Octapeptide de formule D-Phé-Cys-Tyr-D-Trp-Lys-Val-Cys-bêta-Nal-NH2, ou un de ses sels pharmaceutiquement acceptables, à utiliser en thérapeutique médicale.

3. Utilisation d'un octapeptide de formule 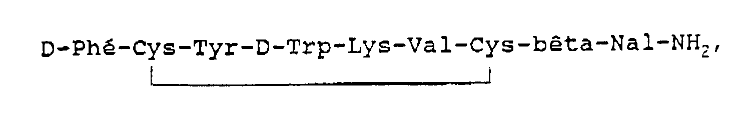
D-Phé-Cys-Tyr-D-Trp-Lys-Val-Cys-bêta-Nal-NH2,

 dans la préparation d'un agent thérapeutique.

4. Utilisation d'un composé suivant la revendication 3, dans laquelle l'activité thérapeutique est l'inhibition de la libération ou de la sécrétion de l'hormone de croissance, de l'insuline, du glucagon, le traitement du cancer, de l'acromégalie, des ulcères, de la pancréatite, de la diarrhée, du diabète, de la cirrhose, de la maladie d'Alzheimer, de l'intoxication par les champignons ou de la douleur en tant qu'analgésique.

5. Composition pharmaceutique comprenant un octapeptide de formule D-Phé-Cys-Tyr-D-Trp-Lys-Val-Cys-bêta-Nal-NH2, ou un de ses sels pharmaceutiquement acceptables, mélangé à un excipient pharmaceutiquement acceptable.

## Revendications (Revendications pour l'(les) Etat(s) contractant(s) suivant(s): ES, GR)

1. Procédé de préparation d'un octapeptide de formule D-Phé-Cys-Tyr-D-Trp-Lys-Val-Cys-bêta-Nal-NH2, ou d'un de ses sels pharmaceutiquement acceptables, le procédé impliquant l'assemblage des acides aminés successifs.

2. Procédé de préparation d'une composition pharmaceutique, le procédé comprenant le mélange d'un octapeptide de formule générale 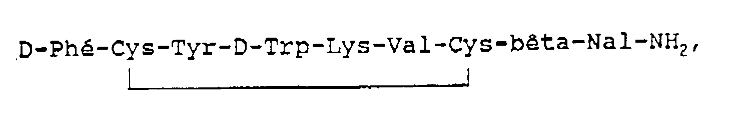
D-Phé-Cys-Tyr-D-Trp-Lys-Val-Cys-bêta-Nal-NH2,

 ou d'un de ses sels pharmaceutiquement acceptables, avec un excipient pharmaceutiquement acceptable.

3. Utilisation d'un octapeptide de formule D-Phé-Cys-Tyr-D-Trp-Lys-Val-Cys-bêta-Nal-NH2, ou d'un de ses sels pharmaceutiquement acceptables, dans la préparation d'un agent thérapeutique.

4. Utilisation d'un composé suivant la revendication 3, dans laquelle l'activité thérapeutique est l'inhibition de la libération ou de la sécrétion de l'hormone de croissance, de l'insuline, du glucagon, le traitement du cancer, de l'acromégalie, des ulcères, de la pancréatite, de la diarrhée, du diabète, de la cirrhose, de la maladie d'Alzheimer, de l'intoxication par les champignons ou de la douleur en tant qu'analgésique.
